# EUROPEAN PATENT SPECIFICATION

(11) **EP 1 590 014 B1**
(45) Date of publication and mention of the grant of the patent: **19.03.2014**
(21) Application number: 04701642.3
(22) Date of filing: 13.01.2004
(51) Int. Cl.: A61M 1/16, A61M 1/36, F16L 37/02, A61M 39/10

(54) **INTEGRATED MODULE FOR BLOOD TREATMENT AND ASSEMBLY PROCESS**
INTEGRIERTES MODUL FÜR DIE BLUTBEHANDLUNG UND VERFAHREN ZUR HERSTELLUNG
MODULE INTEGRE EN VUE D'UN TRAITEMENT SANGUIN ET PROCEDE D'ASSEMBLAGE

(30) Priority: 07.02.2003 IT MI20030214
(43) Date of publication of application: 02.11.2005
(60) Divisional application: 10011559.1
(73) Proprietor: Gambro Lundia AB, 22 643 Lund (SE)
(72) Inventor: DUCHAMP, Jacques, F-69500 Bron (FR); ABERKANE, Aziz, F-69150 Decines (FR); POUCHOULIN, Dominique, F-01390 Tramoyes (FR); MEYSSONNIER, Gabriel, F-38460 Dizimieu (FR)
(74) Representative: Ponzellini, Gianmarco
(86) International application number: PCT/IB2004/000055
(87) International publication number: WO 2004/069309

(56) References cited:
- EP-A- 0 611 227
- EP-A- 0 887 100
- WO-A-01/08722
- DE-A- 2 907 832
- US-A- 4 436 620

## Description

The invention relates to an integrated module for blood treatment and to a manufacturing process for an integrated module for blood treatment.

As is known, for carrying out extracorporeal blood treatments, such as, for example, hemodialysis, hemofiltration, hemodiafiltration, plasmapheresis, extracorporeal blood oxygenation, extracorporeal blood filtration or other treatments, there must be present at least one extracorporeal circuit through which the blood is made to circulate in order to be transported towards a treatment device. The treated blood is then returned to the patient's cardiovascular system.

With reference, by way of example, to a dialysis treatment, the extracorporeal circuit used comprises: a dialysis filter constituted by a container body including at least a first and a second chamber, separated from each other by a semipermeable membrane, a blood withdrawal line leading to the first chamber of the dialyzer filter and a blood return line destined to receive blood outletting from the first chamber and to return it to the patient. The second chamber of the dialyzer filter is connected to a dialysis liquid circulation circuit destined to receive the impurities present in the blood, as well as the excess fluid which is to be removed from the patient's blood.

At present, in extracorporeal blood treatment apparatus, the totality of lines destined for dialysis liquid circulation is housed inside the dialysis machine, while the lines forming the extracorporeal blood circuit are changed after each single treatment and are connected to the dialyzer filter, which can be changed either at each treatment or periodically, according to needs.

From the structural point of view, the dialyzer filter, the dialysis liquid circulation lines and the lines forming the patient's blood withdrawal and return branches are constituted by separate parts which are connected up and cooperate operatively following assembly.

The market offers apparatus, in particular destined for intensive kidney failure treatment, which are advantageously provided with integrated modules comprising a support structure, a dialyzer filter constrained to the support structure by means of a support element emerging from the support structure, as well as a hydraulic circuit comprising the tubing necessary for defining the withdrawal branch and the return branch of the blood from and to the patient, the lines (if present) for infusion of anticoagulant, or of substitution liquids, the dialysis liquid supply line, the discharge line for discharge liquid outletting from the dialysis filter second chamber.

The above-described integrated modules enable an easy and immediate attachment of the lines on the treatment apparatus and do not need any connection between the treatment device, for example a dialyzer filter, and the various tubes or lines destined to carry blood and other fluids. Further, the integrated modules enable removal both of the tubes carrying the blood and those carrying other fluids once the treatment has been concluded. In other words, with a simple loading operation and a connecting-up of the terminals and the fluid transport lines to the relative sources, i.e. bags or other, the user can start up the dialysis apparatus.

Similarly, once the treatment has been concluded, a small number of disconnecting operations and dismounting of the integrated module from the blood treatment machine will enable the operator to completely remove the extracorporeal circuit, the blood treatment device, any tubing for circulation of infusion liquids as well as for the dialysis liquid.

The ease with which the module can be set up guarantees efficiency and speed, much to be appreciated in the case of intensive treatment, where the personnel involved, not necessarily expert in the use of blood treatment machines, can operate quickly and extremely reliably.

Though the above-described integrated modules have had a notable market success, they have shown themselves to be susceptible to improvement in various aspects.

Firstly, in the prior art, the connection between the support body and the blood treatment device includes an additional support interpositioned between the body of the treatment device and the support element, considerably complicating the overall structure of the integrated model.

The presence of an intermediate support structure between the support body and the dialyzer body causes the integrated module to be considerably unwieldy.

Additionally, the need to connect the dialyzer filter or another treatment device used with the extracorporeal blood circuit lines and the treatment fluid lines constitutes a further difficulty, as the connecting-up operations have to be performed in a zone which is difficult to access.

The above has obviously hampered the possibility of automating the assembly stages, considerably increasing production costs of the integrated modules at present on the market.

WO 01/08722 **discloses a support element for an integrated module for blood treatment, comprising a base body and a first and a second connector associated to the base body and distanced one from another. The connectors are directly constrained to the base body and are arranged on a side of the base body. Each connector affords a fluid passage having a first end portion. The connectors and the base body are made of rigid material.**

EP-A-0 887 100 **discloses a support element for an integrated module for blood treatment, comprising a base body and a first and a second connector associated to the base body and distanced one from another. The base body comprises a front wall and a perimeter wall which is adapted to define a work area within which at least a portion of a fluid distribution circuitry can be housed.**

### SUMMARY OF THE INVENTION

A main aim of the present invention is to make available an integrated module for blood treatment comprising a support element, which overcome all of the above-described limitations and drawbacks.

These aims and more besides will better emerge from the detailed description that follows, of an integrated module for blood treatment, as characterized in one or more of the appended claims.

Further characteristics and advantages will better emerge from the detailed description that follows of some preferred embodiments of an integrated module incorporating the support element of the present invention.

The following detailed description will also illustrate a manufacturing process of an integrated module for blood treatment, according to the invention.

### BRIEF DESCRIPTION OF THE DRAWINGS

The detailed description will now be made with reference to the accompanying figures of the drawings, provided as a non-limiting example, in which:
- Figure 1 is a perspective view of a support element for an integrated module according to a first embodiment;
- Figure 2 shows the support element of figure 1 in an upturned position relative to the position of figure 1;
- Figure 3 is a perspective view of a cover for closing an open side of the support element of figure 1;
- Figure 4 is a perspective view of a support element for an integrated module in a second embodiment;
- Figure 5 shows the support element of figure 4 in an upturned position relative to the position of figure 4;
- Figure 6 is a perspective view of an integrated module for extracorporeal treatment of blood with the support element of figure 5;
- Figure 7a shows a detail of the module of figure 6, relating to a coupling between a seating of the support element and a corresponding connector of a blood treatment device;
- Figure 7b shows a detail of figure 1;

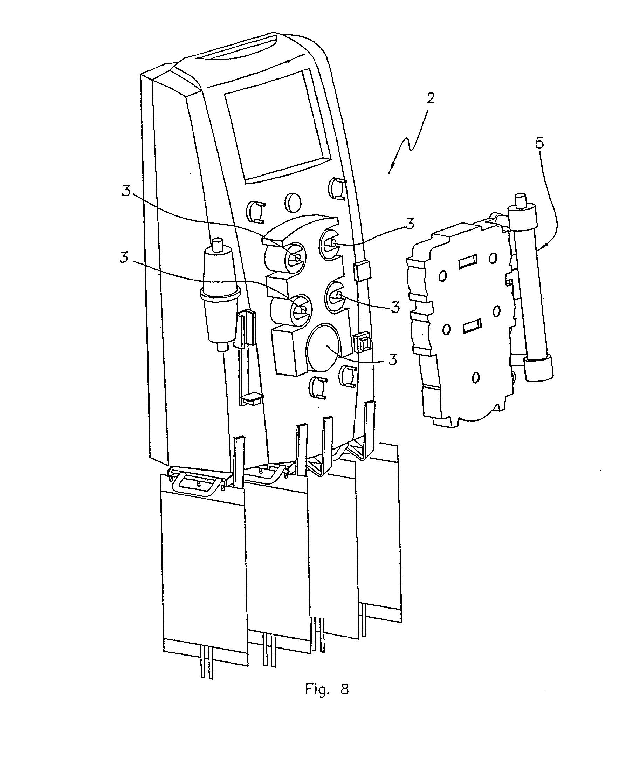

- Figure 8 schematically illustrates the module of figure 6 constrained on a frontal operative wall of a machine for extracorporeal blood treatment;
- Figure 9 is a schematic representation of the module of figure 6 in CRRT (continuous renal replacement therapy) configuration.

### DETAILED DESCRIPTION

With reference to the accompanying figures of the drawings, 1 denotes in its entirety an integrated module for blood treatment. The module 1 can be engaged to a machine for extracorporeal blood treatment 2, provided with one or more pumps 3 destined to cooperate with the module 1. The module 1 comprises a support element 4 to which a blood treatment device 5, for example a plasma-filter, a hemodialysis filter, a hemofiltration filter, a filter for hemodiafiltration or a different unit.

In greater detail, the support element 4 comprises a base body 6 exhibiting at least a first and at least a second connector 7 and 8, distanced one from another, destined to receive and connect with corresponding counter-connectors 9 and 10 of the blood treatment device 5. The first and second connectors 7 and 8 are directly constrained to the base body 6; in the illustrated embodiments the connectors 7 and 8 are made of a rigid plastic material and in a single piece with the base body 6.

The support element 4 exhibits a third connector 11, distanced from the connectors 7, 8 and directly constrained on the base body 6; in the illustrated embodiments the third connector 11 is made of rigid plastic material and in a single piece with the base body 6; the three connectors define pairs of connectors having differentiated interaxes for engaging with corresponding pairs of counter-connectors associated to different blood treatment devices which are mountable on the support element 4. This is so that a single base body 6 can be used to realize integrated modules having different characteristics, thanks to the possibility of engaging treatment devices 5 which are not only different as regards the membrane, but also in terms of overall size and therefore interaxes of the relative counter-connectors.

Each of the connectors 7, 8, 11 constitutes a rigid support and defines a fluid passage having a first end portion 12, destined to be placed in fluid communication with a corresponding channel 13 present in a respective counter-connector 9, 10 exhibited by the blood treatment device 5; each connector 7, 8, 11 also exhibits a second end portion 14, destined to be placed in fluid communication with a fluid distribution circuitry 15 associable to the base body 6. In a further structural detail, each of the connectors 7, 8, 11 comprises a tubular channel 16, defining the first end portion 12, a sealing collar 17, in a position which is radially external to the tubular channel 16, and a connecting wall 18, which develops continuously between an external lateral surface 19 of the tubular channel 16 and an internal lateral surface 20 of the sealing collar 17.

The external lateral surface 19 of the tubular channel 16, the internal lateral surface 20 of the sealing collar 17 and the connecting wall 18 together define an annular seating 21, a bottom of which is delimited by the connecting wall 18, shaped in order to receive and engage a corresponding counter-connector of the blood treatment device.

The tubular channel 16 is coaxially arranged with respect to the sealing collar 17, and has geometry of revolution there-with, with a common axis of symmetry. The annular seating 21 exhibits an increasing radial dimension as it progresses from the bottom connecting wall 18; it comprises a first zone 22, adjacent to the bottom and having a constant radial dimension; a second zone 23, distal with respect to the bottom and having a constant radial dimension which is greater than the radial dimension of the first zone 22; and a third zone 24, which is a transit zone between the first and second zones 22 and 23 and which has a progressively growing radial dimension as it progresses away from the bottom connecting wall 18.

The tubular channel 16 and the sealing collar 17 of each connector 7, 8, 11 are parallel to one another in the base body 6, defining a single coupling direction with the corresponding counter-connectors of a treatment device 5.

In the illustrated embodiments, the various counter-connectors and connectors exhibit axes of symmetry which are perpendicular to a frontal wall 25 of the support element 4.

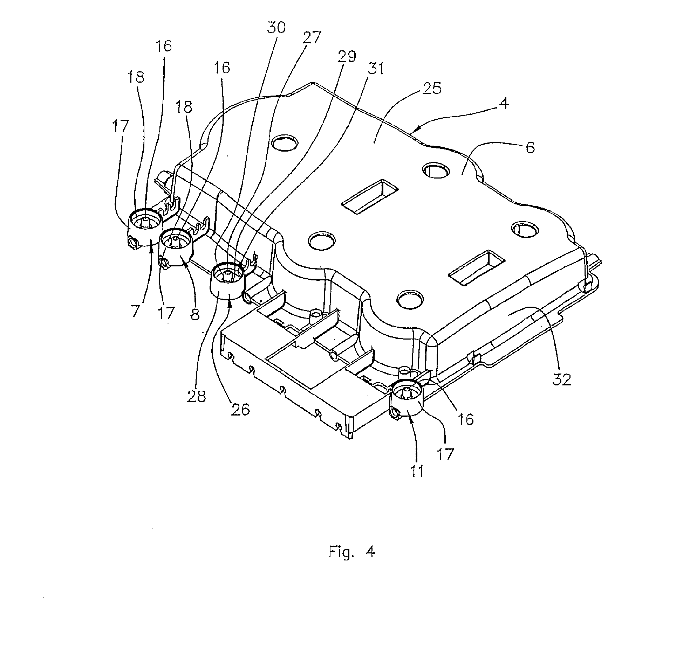

The support element 4 shown in figures 4-7 further comprises a fourth connector 26 which is distanced from the first, second and third connectors 7, 8 and 11. The fourth connector 26 is also directly connected to the support element 4.

In the embodiment of figures 4-7 the fourth connector 26 is made of rigid plastic in a single piece with the base body 6 and defines, with at least one of the other connectors 7, 8 and 11 a further pair of connectors which can be engaged to a corresponding pair of counter-connectors associated to a blood treatment device mountable to the support element 4.

The fourth connector 26 comprises a central cylindrical body 27 for positioning, a sealing collar 28 located in a radially external position with respect to the central cylindrical body 27, and a bottom connecting wall 29 which develops continuously between an external lateral surface 30 of the central cylindrical body 27 and an internal lateral surface 31 of the collar 28. The fourth connector 26 defines a connecting and sealing site for a counter-connector of the blood treatment device 5.

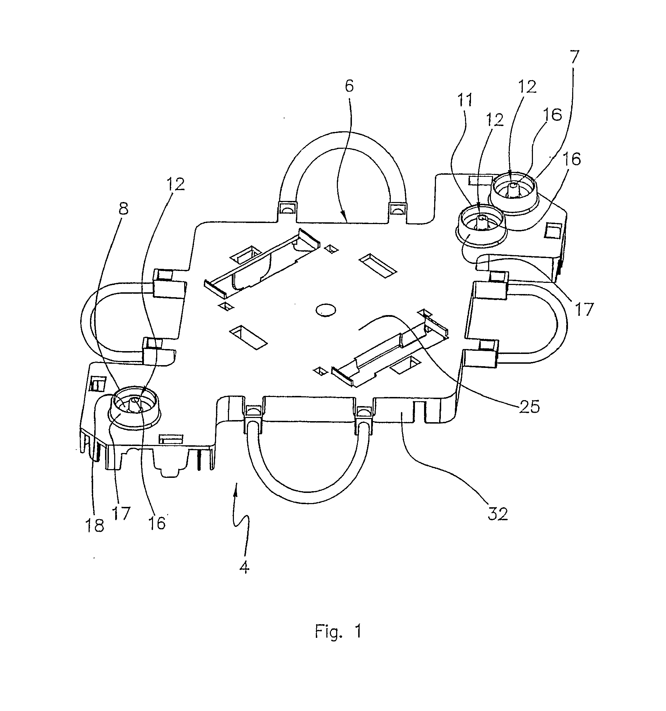

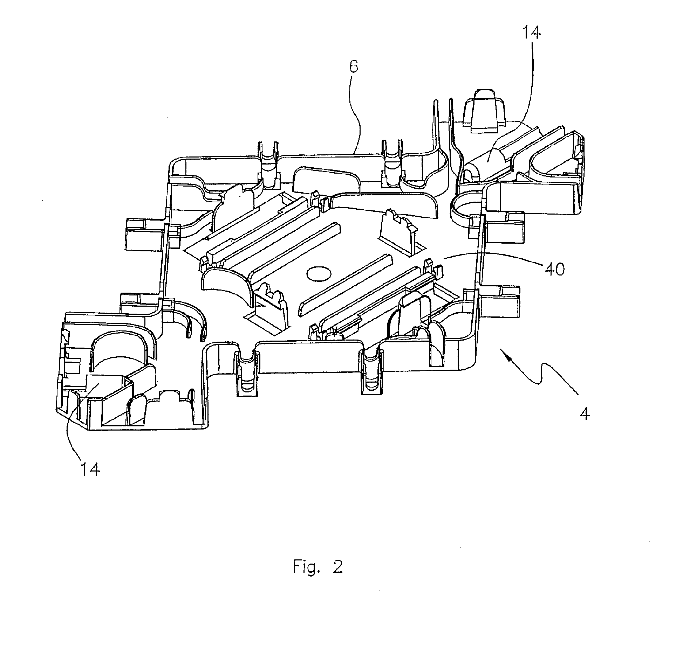

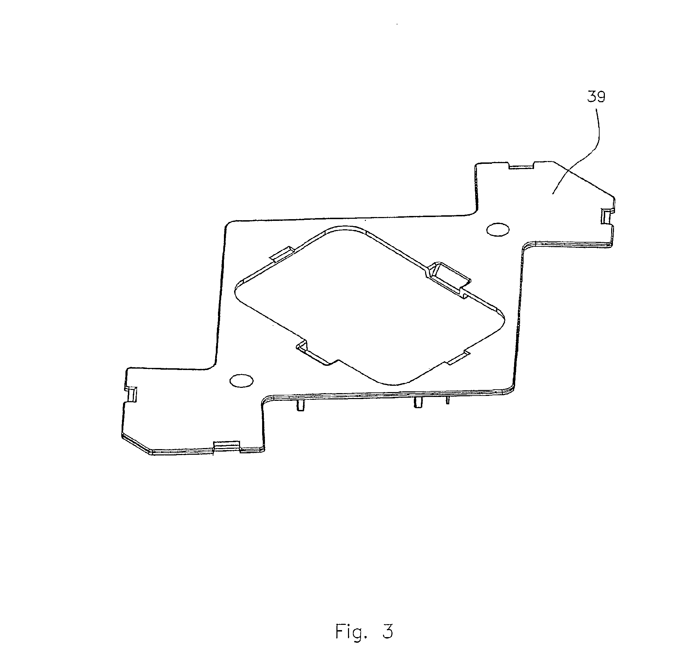

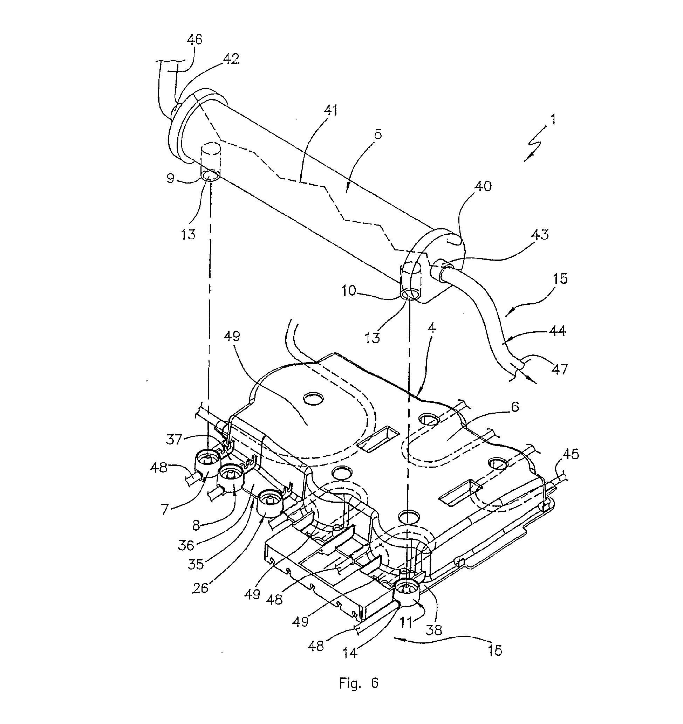

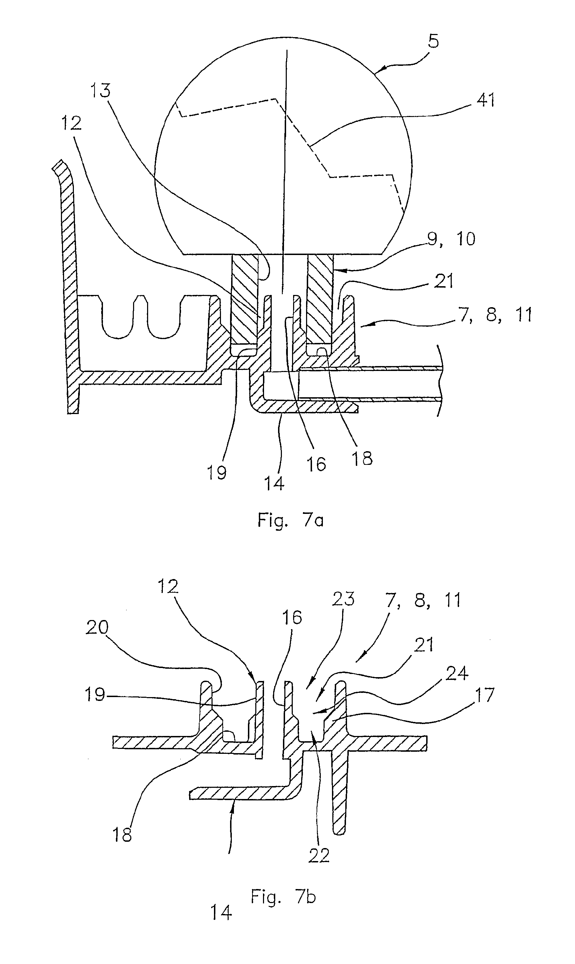

As shown in figures 6, 7a (and the same goes for the support elements of figures 1-3, 7b), the various connectors are made of rigid material in order to offer a mechanical support to the blood treatment device and, according to each individual case, to define a passage or an obstruction for fluid passing through the counter-connectors 9, 10.

In the support element of figures 4-6 the four connectors are aligned and arranged on one side of the base body 6. More precisely, the base body 6 of the element illustrated in figures 4-6 and 7a comprises a frontal wall 25 and a perimeter wall 32 connected around an edge thereof to the frontal wall 25, which together define a works housing area 33 which can house at least a portion of the works of the support element, i.e. a fluid distribution circuitry 15 destined to be associated to the support element 4.

The works housing area 33 exhibits an open side 34 which enables the integrated module 1 to be correctly positioned and adequately locked onto the machine, as will be better described herein below.

The support element 4 exhibits an auxiliary structure 35 which extends laterally and externally with respect to the works housing area 33 from a base zone 36 of the perimeter wall 32. The four connectors emerge from the auxiliary structure 35: the first, second and fourth connector 7, 8, 26 are adjacently situated, and arranged at a first end zone 37 of the auxiliary structure 35, while the third connector 11 is located at a second end zone 38, opposite the first end zone 37.

In the illustrated embodiment of figures 1-3, the base body 6 comprises a frontal wall 25 and a perimeter wall 32 joined at an edge thereof to the frontal wall 25, defining a works housing area 33 which can house at least a portion of the fluid distribution circuitry 15 destined to be associated to the support element 4.

In this embodiment, however, the connectors 7, 8 and 11 are not aligned and emerge directly from the frontal wall 25. Further, a cover 39 is associated to the perimeter wall 32 on an opposite edge thereof with respect to the frontal wall 25.

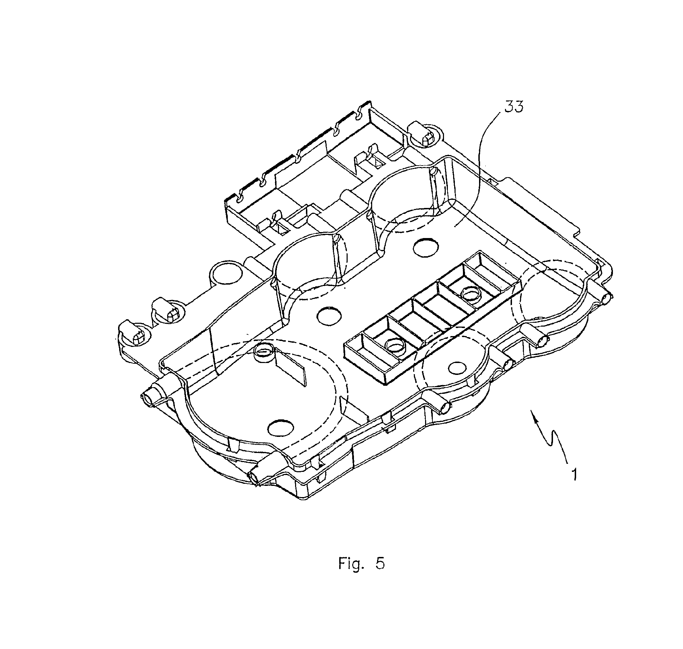

A support element according to the description can advantageously be used for realizing an integrated module, such as for example the module illustrated in figure 6, where by way of example the support element 4 of figures 4 and 5 is used.

As can be observed, the blood treatment device 5 is fixed to the support element 4 by at least one pair of connectors; the blood treatment device comprises a body 40, at least one semipermeable membrane 41 (for example a parallel hollow fiber membrane or a plate membrane) operating internally of the body 40 and defining a first chamber and a second chamber; a first and a second connector are associated to the body 40 and fixed to the respective connectors on the base body 6.

The first and second counter-connectors 9, 10, are tubular and are in fluid communication with the second chamber of the treatment device and with respective first end portions 12 of the connectors.

The treatment device exhibits an inlet port 42 to the first chamber, and at least one outlet port 43 from the first chamber, for connection of an extracorporeal blood circuit line 44 or another physiological fluid.

A fluid distribution circuitry 15 is attached to the support element 4 and cooperates with the treatment device 5. In more detail, the circuitry comprises:
- at least one discharge line 45 of discharge fluid, in communication with the second terminal portion 14 of one of the connectors;
- at least one blood line 44 having a blood withdrawal branch 46, placed in communication with the inlet 42 of the first chamber, and at least one branch 47 of a blood return line, placed in communication with the outlet 43 of the first chamber;
- at least one supply line 48 of fresh dialysis liquid, placed in communication with the second end portion 14 of another of the connectors.

Each of the lines is constrained to the support element 4, defining at least one tract of tubing 49 which is arranged in a U-shape, in relation to the support element 4.

During operation the U-shaped tracts are destined to cooperate with the respective peristaltic pumps 3 located on a panel of a machine for extracorporeal blood treatment. Each tract of U-shaped tubing extends internally or externally (figure 7) with respect to the perimeter wall 32 of the support element 4.

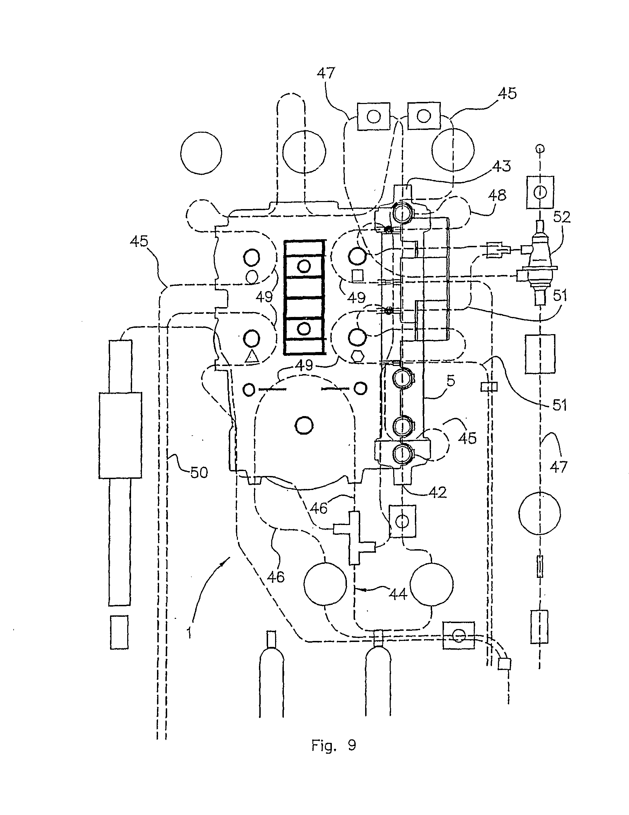

Figure 9 is a diagram of the integrated module 1 in the CRRT configuration. As can be observed, the module 1 also has lines 50 and 51 for respective pre and post blood pump infusions (predilution and/or post-dilution).

An air separator device 52 operates on the branch 47 of the blood line 44 and receives an infusion line 51.

The invention also relates to an assembly procedure for an integrated module for fluid treatment which comprises the stages of predisposing a support element 4, for example such as in figures 1-3 or in figures 4-6, as well as a treatment device 5 which is intended for coupling to the support element 4. The blood treatment device 5 is then fixed to the support element 4. Finally a fluid distribution circuitry 15 is associated to the support element 4 and to the blood treatment device 5 so as to create the necessary blood circulation lines, the discharge lines, the infusion lines for any liquid substitution lines, and dialysis lines.

The connection of the distribution circuitry to the blood treatment device can be done before, at the same time as or after the circuitry fixing stage to the support element 4.

The fixing stage of the blood treatment device to the support element 4 comprises the sub-stages of selecting a pair of connectors to which the counter-connectors 9, 10 on the blood treatment device are to be connected, applying a predetermined quantity of glue, normally polymer-resin based, in the annular seatings 21 of each chosen connector, at least partially inserting each counter-connector in the respective annular seating in order to obtain a mechanical bond and a liquid-proof seal coupling.

During the insertion stage, at least a portion of the glue applied in the annular seating actually settles in the second zone 23 of the annular seating. On completion of the counter-connector insertion stage in the annular seating, the volume of the quantity of glue previously applied added to the volume of the portion of counter-connector housed in the annular seating is less than the overall volume of the annular seating. This prevents any glue material from migrating towards the tubular channel 16 and causing a partial or total occlusion.

The stage of associating a fluid distribution circuitry 15 to the support element 4 and the blood treatment device 5 comprises the sub-stages of liquid-proof sealing of an end portion of a discharge fluid discharge line 45 with the second end portion 14 of one of the connectors, and of sealedly fixing an end portion of a fresh dialysis liquid supply line 48 to the second end portion of a further of the connectors.

The stage of associating the blood distribution circuitry also includes sealedly fixing an end portion of a blood withdrawal branch 46 to an inlet port of the first chamber, and an end portion of a blood return branch 47 to an outlet port of the first chamber.

The fixing of the various above-mentioned end portions can be achieved by gluing, friction fitting or hot-coupling.

The invention provides important advantages.

Firstly, the direct fixing of the blood treatment device to the selected connectors of the support element does not require the use of other support elements of the same device.

Further, the connectors receive on one side the counter-connectors of the blood treatment device and on the other side the end portions of distribution circuitry lines, realizing a contemporaneous mechanical and hydraulic connection between the fluid distribution circuitry and the blood treatment device.

The presence of various connectors means the treatment device can be used with connectors having different interaxes.

The special fixing modality of the blood treatment device and the various fluid lines to the support element considerably facilitates the assembly process of an integrated module according to the invention.

The specific structure of the integrated module and the support element minimises the length of fluid line needed to realize the connections with the blood treatment device.

## Claims

1. An assembly process for an integrated module (1) for fluid treatment comprising stages of:
- predisposing a support element (4) for an integrated module (1) for blood treatment, comprising:
o a base body (6);
o at least a first and at least a second connector (7) and (8) associated to the base body (6) and distanced one from another, destined to receive and engage with corresponding counter-connectors (9) and (10) of a blood treatment device (5) which is mountable on the support element (4);
o at least a third connector (11), distanced from the said first connector (7) and from the said second connector (8) and directly constrained to the base body (6), the said first, second and third connectors (7), (8) and (11) defining pairs of connectors having differentiated interaxes there-between for engaging to corresponding pairs of counter-connectors (9) and (10) associated to various blood treatment devices which are mountable on the support element (4);
- fixing a blood treatment device (5) to the support element (4);
- associating a fluid distribution circuitry (15) to the support element (4) and to the blood treatment device (5), **characterized in that** the step of fixing the blood treatment device comprises the sub-step of:
o selecting of a pair of connectors (7), (8), (11) to which the counter-connectors (9), (10) of the blood treatment device (5) are to be fixed;
o depositing a prefixed quantity of glue in the annular seatings (21) of each connector which has been selected;
o at least partially inserting each counter-connector (9), (10) into a respective annular seating (21) in order to obtain a mechanical lock and a liquid-proof seal.

2. The process of claim 1, wherein during the said insertion stage, at least one portion of the prefixed quantity of glue is arranged in the said second zone (23) of the respective annular seating (21).

3. The process of claim 2, wherein at an end of the said insertion stage, a volume of the said prefixed quantity of glue added to a volume of the portion of counter-connector (9), (10) housed in the annular seating (21) is less than a total volume of the annular seating.

4. The process of claim 1, wherein the stage of associating a fluid distribution circuitry (15) to the support element (4) and to the blood treatment device (5) comprises sub-stages of:
- liquid-proof fixing of an end portion of a discharge line (45) of a discharge fluid with the second end portion (14) of one of the said connectors (7), (8), (11), (26);
- sealedly fixing an end portion of a fresh dialysis liquid supply line (48) to the second end portion (14) of another of the said connectors (7), (8), (11), (26);
- sealedly fixing an end portion of a blood withdrawal branch (46) to the inlet port (42) of the first chamber, and an end portion of a blood return branch (47) to the outlet port (43) of the first chamber.

5. An integrated module (1) for fluid treatment, comprising:
- a support element (4) for an integrated module (1) for blood treatment, comprising:
o a base body (6);
o at least a first and at least a second connector (7) and (8) associated to the base body (6) and distanced one from another, destined to receive and engage with corresponding counter-connectors (9) and (10) of a blood treatment device (5) which is mountable on the support element (4);
o at least a third connector (11), distanced from the said first connector (7) and from the said second connector (8) and directly constrained to the base body (6), the said first, second and third connectors (7), (8) and (11) defining pairs of connectors having differentiated interaxes there-between for engaging to corresponding pairs of counter-connectors (9) and (10) associated to various blood treatment devices which are mountable on the support element (4);
- at least one blood treatment device (5) engaged on the support element (4), said blood treatment device (5) comprising:
o a containment body (40);
o at least one semi-permeable membrane (41) operating internally of the containment body (40) and defining a first chamber and a second chamber;
o a first counter-connector (9) and a second counter-connector (10) associated to the containment body (40) and glue fixed to a pair of the three connectors (7), (8) and (11) associated to the base body (6), at least one of the first counter-connector and the second counter-connector being placed in fluid communication with the second chamber of the blood treatment device (5) and with respective first end portions (12) of the said connectors;
o at least one inlet port (42) to the first chamber; and
o at least one outlet port (43) from the first chamber;
- a fluid distribution circuitry (15) associated to the support element (4) and cooperating with the blood treatment device (5).

6. The integrated module (1) of claim 5, wherein the first and the second connectors (7) and (8) are directly constrained to the base body (6).

7. The integrated module (1) of claim 6, wherein the first and second connectors (7) and (8) are made in a single piece with the base body (6).

8. The integrated module (1) of claim 5, wherein the third connector (11) is made in a single piece with the base body (6).

9. The integrated module (1) of any one of the preceding claims 5 to 8, wherein each of the said connectors (7), (8) and (11) affords a fluid passage having a first end portion (12), destined to be placed in fluid communication with a corresponding channel (13) in a respective counter-connector (8) and (9) on the blood treatment device (5), and a second end portion (14), destined to be placed in fluid communication with a fluid distribution circuitry (15) associable to the base body (6).

10. The integrated module (1) of claim 9, wherein each of the said connectors (7), (8) and (11) comprises:
- a tubular channel (16), defining the said first end portion (12),
- a sealing collar (17), set in a radially external position with respect to the tubular channel (16), and
- a connecting wall (18), developing continuously between an external lateral surface (19) of the said tubular channel (16) and an internal lateral surface (20) of the said sealing collar (17) to define an annular seating (21) for engagement of each counter-connector (9) and (10).

11. The integrated module (1) of claim 10, wherein the tubular channel (16) defining the said first end portion (12) is coaxially arranged with respect to the sealing collar (17), the said annular seating (21) exhibiting a bottom which is delimited by the said connecting wall (18).

12. The integrated module (1) of claim 11, wherein the said annular seating (21) exhibits a radial dimension which increases progressively in a direction moving away from the said bottom wall (18).

13. The integrated module (1) of claim 12, wherein the said annular seating (21) exhibits: a first zone (22), adjacent to the said bottom wall (18) and having a constant radial dimension; a second zone (23), distal of the said bottom wall (18) and having a constant radial dimension which is greater than the radial dimension of the first zone (22); and a third zone (24), which is a transition zone between the first zone (22) and the second zone (23) and has a progressively increasing dimension in a distancing direction from the said bottom wall (18).

14. The integrated module (1) of claim 10, wherein the tubular channel (16) and the sealing collar (17) of each connector (7), (8) and (11) are parallel to one another as they emerge from the base body (6), defining a single coupling direction for coupling with corresponding counter-connectors (9) and (10) of a blood treatment device (5).

15. The integrated module (1) of claim 5, comprising a fourth connector (26), distanced from the said first, second and third connectors (7), (8) and (11), which fourth connector (26) is made in a single piece with the base body (6) and defines, with at least one of the said first, second and third connectors (7), (8) and (11), a further pair of connectors which can be engaged to a corresponding pair of counter-connectors associated to a blood treatment device which is mountable on the support element.

16. The integrated module (1) of claim 15, wherein the fourth connector (26) comprises:
- a central cylindrical positioning body (27);
- a sealing collar (28), set in a radially external position to the cylindrical positioning body (27); and
- a connecting wall (29), developing continuously between an external lateral surface (30) of the said cylindrical positioning body (27) and an internal lateral surface (31) of the said sealing collar (28);
the said fourth connector (26) defining a connecting and sealing site for a counter-connector of the blood treatment device.

17. The integrated module (1) of claim 6 or 15, wherein the said connectors (7), (8), (11) and (26) and the said base body (6) are made of a rigid material in order to offer a mechanical support for the blood treatment device.

18. The integrated module (1) of claim 15, wherein the said connectors (7), (8), (11) and (26) are aligned one to another.

19. The integrated module (1) of claim 6 or 15, wherein the said connectors (7), (8), (11) and (26) are arranged on a side of the base body (6).

20. The integrated module (1) of claim 5, wherein the said base body (6) comprises a frontal wall (25) and a perimeter wall (32), which perimeter wall (32) is connected by a side thereof to the frontal wall (25) and defines a works area (33) within which at least a portion of a fluid distribution circuitry (15) destined to be associated to the support element (4) can be housed.

21. The integrated module (1) of claim 20, comprising an auxiliary structure (35) extending laterally and externally with respect to the said works housing area (33) from a base zone (36) of the perimeter wall (32), the said connectors (7), (8), (11) and (26) emerging from the said auxiliary structure (35).

22. The integrated module (1) of claim 5, wherein the said connectors (7), (8) and (11) are not aligned one to another.

23. The integrated module (1) of claim 5, wherein the base body (6) comprises a frontal wall (25), from which the said connectors (7), (8) and (11) directly project, and a cover (39), associated to a perimeter wall (32) at an opposite edge thereof with respect to the frontal wall (25).

24. The integrated module of any one of the preceding claims 5 to 23, wherein the said blood treatment device (5) is fixed to the base body (6) by at least a pair of the said connectors (7), (8) and (11).

25. The integrated module of claim 5, wherein the said pair of connectors is interpositioned between the counter-connectors (9) and (10) and a portion of the fluid distribution circuitry (15).

26. The integrated module of claim 5, wherein the fluid distribution circuitry (15) comprises at least one discharge line (45) of a discharge fluid, placed in communication with the second end portion (14) of one of the said connectors (7), (8) and (11).

27. The integrated module of claim 5, wherein the fluid distribution circuitry (15) comprises at least one fresh dialysis liquid supply line (48), placed in communication with the second end portion (14) of another of the connectors (7), (8) and (11).

28. The integrated module of any one of the preceding claims 5 to 27, wherein the fluid distribution circuitry (15) comprises at least one blood circuit line (44) having a blood withdrawal branch (46), placed in communication with the inlet port (42) of the first chamber, and at least one blood return branch (47), placed in communication with the outlet port (43) of the first chamber.

29. The module of any one of claims 26, 27 or 28, wherein at least one of the said lines (44), (45) and (48) is constrained to the support element (4), defining at least one tract of tubing (49) which is U-shaped in relation to the support element (4) and which is destined during operation to cooperate with a peristaltic pump (3).

30. The integrated module of claim 29, wherein the at least one U-shaped tract of tubing (49) extends internally or externally with respect to the perimeter wall (32) of the support element (4).

## Patentansprüche

1. Montageverfahren für ein integriertes Modul (1) zur Fluidbehandlung umfassend die Phasen:
- Vorbereiten eines Tragelementes (4) für ein integriertes Modul (1) zur Blutbehandlung, umfassend:
○ einen Basiskörper (6);
○ mindestens einen ersten und mindestens einen zweiten Verbinder (7) und (8), die mit dem Basiskörper (6) verbunden und voneinander beabstandet sind, vorgesehen zum Empfangen und Eingreifen mit entsprechenden Gegenverbindern (9) und (10) einer Blutbehandlungsvorrichtung (5), die am Tragelement (4) montierbar ist;
○ mindestens einen dritten Verbinder (11), der vom ersten Verbinder (7) und vom zweiten Verbinder (8) beabstandet und mit dem Basiskörper (6) unmittelbar fest verbunden ist, wobei die ersten, zweiten und dritten Verbinder (7), (8) und (11) Verbinderpaare definieren, die unterschiedliche Zwischenachsen haben zum Eingreifen mit entsprechenden Paaren von Gegenverbindern (9) und (10), die mit verschiedenen, am Tragelement (4) montierbaren Blutbehandlungsvorrichtungen verbunden sind;
- Befestigen einer Blutbehandlungsvorrichtung (5) am Tragelement (4);
- Verbinden eines Fluidverteilungskreislaufs (15) mit dem Tragelement (4) und mit der Blutbehandlungsvorrichtung (5), **dadurch gekennzeichnet, dass** der Schritt des Befestigens der Blutbehandlungsvorrichtung die folgenden Unterschritte umfasst:
○ Auswählen eines Paares von Verbindern (7), (8), (11), an den die Gegenverbinder (9), (10) der Blutbehandlungsvorrichtung (5) zu befestigen sind;
○ Auftragen einer vorbestimmten Menge von Klebemittel in den Ringauflagen (21) jedes ausgewählten Verbinders;
○ mindestens teilweise Einführen jedes Gegenverbinders (9), (10) in eine entsprechende Ringauflage (21), um eine mechanische Verriegelung und eine Flüssigkeitsabdichtung zu erzielen.

2. Verfahren nach Anspruch 1, wobei während des Einführungsschrittes mindestens ein Teil der vorbestimmten Menge von Klebemittel im zweiten Bereich (23) der entsprechenden Ringauflage (21) aufgetragen wird.

3. Verfahren nach Anspruch 2, wobei am Ende des Einführungsschrittes ein Volumen der vorbestimmten Menge von Klebemittel zuzüglich eines Volumens des Abschnitts des Gegenverbinders (9), (10), der in der Ringauflage (21) aufgenommen ist, weniger als ein Gesamtvolumen der Ringauflagen beträgt.

4. Verfahren nach Anspruch 1, wobei die Phase des Verbindens eines Fluidverteilungskreislaufs (15) mit dem Tragelement (4) und mit der Blutbehandlungsvorrichtung (5) die Unterphasen umfasst:
- flüssigkeitsdichtes Befestigen eines Endabschnitts einer Abflussleitung (45) für ein Abflussfluid am zweiten Endabschnitt (14) eines der Verbinder (7), (8), (11), (26);
- abgedichtetes Befestigen eines Endabschnitts einer Zuführungsleitung für frische Dialyseflüssigkeit (48) am zweiten Endabschnitt (14) eines anderen der Verbinder (7), (8), (11), (26);
- abgedichtetes Befestigen eines Endabschnitts einer Blutentnahmeabzweigung (46) an der Eingangsöffnung (42) der ersten Kammer, und eines Endabschnitts einer Blutrückführungsabzweigung (47) an der Ausgangsöffnung (43) der ersten Kammer.

5. Integriertes Modul (1) zur Fluidbehandlung umfassend:
- ein Tragelement (4) für ein integriertes Modul (1) zur Blutbehandlung umfassend:
○ einen Basiskörper (6);
○ mindestens einen ersten und mindestens einen zweiten Verbinder (7) und (8), die mit dem Basiskörper (6) verbunden und voneinander beabstandet sind, vorgesehen zum Empfangen und Eingreifen mit entsprechenden Gegenverbindern (9) und (10) einer Blutbehandlungsvorrichtung (5), die am Tragelement (4) montierbar ist;
○ mindestens einen dritten Verbinder (11), der vom ersten Verbinder (7) und vom zweiten Verbinder (8) beabstandet und mit dem Basiskörper (6) unmittelbar fest verbunden ist, wobei die ersten, zweiten und dritten Verbinder (7), (8) und (11) Verbinderpaare definieren, die unterschiedliche Zwischenachsen haben zum Eingreifen mit entsprechenden Paaren von Gegenverbindern (9) und (10), die mit verschiedenen, am Tragelement (4) montierbaren Blutbehandlungsvorrichtungen verbunden sind;
- mindestens eine Blutbehandlungsvorrichtung (5), die am Tragelement (4) eingreift, wobei die Blutbehandlungsvorrichtung (5) umfasst:
○ einen Einschließungskörper (40);
○ mindestens eine semipermeable Membran (41), die innen im Einschließungskörper (40) wirkt und eine erste Kammer und eine zweite Kammer definiert;
○ einen ersten Gegenverbinder (9) und einen zweiten Gegenverbinder (10), die mit dem Einschließungskörper (40) verbunden und an ein Paar der drei mit dem Basiskörper (6) verbundenen Verbinder (7), (8) und (11) geklebt sind, wobei mindestens einer des ersten Gegenverbinders und des zweiten Gegenverbinders mit der zweiten Kammer der Blutbehandlungsvorrichtung (5) und mit entsprechenden ersten Endabschnitten (12) der Verbinder in Fluidverbindung gesetzt wird;
○ mindestens eine Eingangsöffnung (42) zur ersten Kammer; und
○ mindestens einen Ausgangsöffnung (43) aus der ersten Kammer;
- einen Fluidverteilungskreislauf (15), der mit dem Tragelement (4) verbunden ist und mit der Blutbehandlungsvorrichtung (5) zusammenwirkt.

6. Integriertes Modul (1) nach Anspruch 5, wobei der erste und der zweite Verbinder (7) und (8) an den Basiskörper (6) unmittelbar gebunden sind.

7. Integriertes Modul (1) nach Anspruch 6, wobei der erste und der zweite Verbinder (7) und (8) einstückig mit dem Basiskörper (6) hergestellt sind.

8. Integriertes Modul (1) nach Anspruch 5, wobei der dritte Verbinder (11) einstückig mit dem Basiskörper (6) hergestellt ist.

9. Integriertes Modul (1) nach einem der vorherigen Ansprüche 5 bis 8, wobei jeder der Verbinder (7), (8) und (11) einen Fluiddurchgang mit einem ersten Endabschnitt (12), der mit einem entsprechenden Kanal (13) in einem entsprechenden Gegenverbinder (8) und (9) an der Blutbehandlungsvorrichtung (5) in Fluidverbindung gesetzt werden soll, und einem zweiten Endabschnitt (14), der mit einem mit dem Basiskörper (6) verbindbaren Fluidverteilungskreislauf (15) in Fluidverbindung gesetzt werden soll, ermöglicht.

10. Integriertes Modul (1) nach Anspruch 9, wobei jeder der Verbinder (7), (8) und (11) umfasst:
- einen Rohrkanal (16), der den ersten Endabschnitt (12) definiert,
- eine Dichtmanschette (17), der in einer radial äußeren Stellung zum Rohrkanal (16) angeordnet ist, und
- eine Verbindungswand (18), die sich kontinuierlich zwischen einer äußeren Seitenfläche (19) des Rohrkanals (16) und einer inneren Seitenfläche (20) der Dichtmanschette (17) entwickelt, um eine Ringauflage (21) zum Eingreifen jedes Gegenverbinders (9) und (10) zu definieren.

11. Integriertes Modul (1) nach Anspruch 10, wobei der Rohrkanal (16), der den ersten Endabschnitt (12) definiert, koaxial gegenüber der Dichtmanschette (17) angeordnet ist, wobei die Ringauflage (21) einen Boden besitzt, der von der Verbindungswand (18) begrenzt wird.

12. Integriertes Modul (1) nach Anspruch 11, wobei die Ringauflage (21) eine radiale Größe aufweist, die in einer Richtung weg von der Bodenwand (18) progressiv zunimmt.

13. Integriertes Modul (1) nach Anspruch 12, wobei die Ringauflage (21) aufweist: einen ersten Bereich (22), der an der Bodenwand (18) anliegt und eine konstante radiale Größe besitzt; einen zweiten Bereich (23), der distal von der Bodenwand (18) liegt und eine konstante radiale Größe besitzt, die größer ist als die radiale Größe des ersten Bereichs (22); und einen dritten Bereich (24), der ein Übergangsbereich zwischen dem ersten Bereich (22) und dem zweiten Bereich (23) ist und eine progressiv zunehmende Größe in einer Richtung weg von der Bodenwand (18) besitzt.

14. Integriertes Modul (1) nach Anspruch 10, wobei der Rohrkanal (16) und die Dichtmanschette (17) jedes Verbinders (7), (8) und (11) zueinander parallel sind, wenn sie aus dem Basiskörper (6) ausgehen, wodurch sie eine einzige Kopplungsrichtung zum Koppeln mit entsprechenden Gegenverbindern (9) und (10) einer Blutbehandlungsvorrichtung (5) definieren.

15. Integriertes Modul (1) nach Anspruch 5, umfassend einen vierten Verbinder (26), der von den ersten, zweiten und dritten Verbindern (7), (8) und (11) beabstandet ist, wobei der vierte Verbinder (26) einstückig mit dem Basiskörper (6) hergestellt ist und mit mindestens einem der ersten, zweiten und dritten Verbinder (7), (8) und (11) ein weiteres Verbinderpaar definiert, das mit einem entsprechenden Paar von Gegenverbindern eingreifen kann, die mit einer am Tragelement montierbaren Blutbehandlungsvorrichtung verbunden sind.

16. Integriertes Modul (1) nach Anspruch 15, wobei der vierte Verbinder (26) umfasst:
- einen zentralen zylindrischen Positionierkörper (27);
- eine Dichtmanschette (28), die in einer radial äußeren Stellung zum zylindrischen Positionierkörper (27) angeordnet ist;
- eine Verbindungswand (29), die sich durchgehend zwischen einer äußeren Seitenfläche (30) des zylindrischen Positionierkörpers (27) und einer inneren Seitenfläche (31) der Dichtmanschette (28) entwickelt;
wobei der vierte Verbinder (26) eine Verbindungs- und Abdichtungsstelle für einen Gegenverbinder der Blutbehandlungsvorrichtung definiert.

17. Integriertes Modul (1) nach Anspruch 6 oder 15, wobei die Verbinder (7), (8), (11) und (26) sowie der Basiskörper (6) aus einem festen Werkstoff bestehen, um eine mechanische Stützte für die Blutbehandlungsvorrichtung anzubieten.

18. Integriertes Modul (1) nach Anspruch 15, wobei die Verbinder (7), (8), (11) und (26) miteinander ausgerichtet sind.

19. Integriertes Modul (1) nach Anspruch 6 oder 15, wobei die Verbinder (7), (8), (11) und (26) an einer Seite des Basiskörpers (6) angeordnet sind.

20. Integriertes Modul (1) nach Anspruch 5, wobei der Basiskörper (6) eine Vorderwand (25) und eine Umfangswand (32) umfasst, wobei die Umfangswand (32) durch eine ihrer Seiten mit der Vorderwand (25) verbunden ist und einen Arbeitsbereich (33) definiert, in dem mindestens ein Abschnitt eines Fluidverteilungskreislaufs (15), der dazu vorgesehen ist, mit dem Tragelement (4) verbunden zu werden, aufgenommen werden kann.

21. Integriertes Modul (1) nach Anspruch 20, umfassend eine Hilfsstruktur (35), die sich in Bezug auf den Arbeitsaufnahmebereich (33) seitlich und außen gegenüber von einem Basisbereich (36) der Umfangswand (32) erstreckt, wobei die Verbinder (7), (8), (11) und (26) aus der Hilfsstruktur (35) herausragen.

22. Integriertes Modul (1) nach Anspruch 5, wobei die Verbinder (7), (8) und (11) nicht miteinander ausgerichtet sind.

23. Integriertes Modul (1) nach Anspruch 5, wobei der Basiskörper (6) eine Vorderwand (25), aus der die Verbinder (7), (8) und (11) unmittelbar vorspringen, und eine Abdeckung (39), die mit einer Umfangswand (32) an einer entgegengesetzten Kante davon gegenüber der Vorderwand (25) verbunden ist, umfasst.

24. Integriertes Modul nach einem der vorherigen Ansprüche 5 bis 23, wobei die Blutbehandlungsvorrichtung (5) am Basiskörper (6) durch mindestens ein Paar der Verbinder (7), (8) und (11) befestigt ist.

25. Integriertes Modul nach Anspruch 5, wobei das Verbinderpaar zwischen den Gegenverbindern (9) und (10) und einen Abschnitt des Fluidverteilungskreislaufs (15) zwischengeordnet.

26. Integriertes Modul nach Anspruch 5, wobei der Fluidverteilungskreislauf (15) mindestens eine Abflussleitung (45) für ein Abflussfluid umfasst, die mit dem zweiten Endabschnitt (14) eines der Verbinder (7), (8) und (11) in Kommunikation gesetzt ist.

27. Integriertes Modul nach Anspruch 5, wobei der Fluidverteilungskreislauf (15) mindestens eine Zuführungsleitung für frische Dialyseflüssigkeit (48) umfasst, die mit dem zweiten Endabschnitt (14) eines anderen der Verbinder (7), (8) und (11) in Kommunikation gesetzt ist.

28. Integriertes Modul nach einem der vorherigen Ansprüche 5 bis 27, wobei der Fluidverteilungskreislauf (15) mindestens eine Blutkreislaufleitung (44) mit einer Blutentnahmeabzweigung (46), die mit der Eingangsöffnung (42) der ersten Kammer in Kommunikation gesetzt ist, und mindestens einer Blutrückführungsabzweigung (47), die mit der Ausgangsöffnung (43) der ersten Kammer in Kommunikation gesetzt ist, umfasst.

29. Modul nach einem der Ansprüche 26, 27 oder 28, wobei mindestens eine der Leitungen (44), (45) und (48) mit dem Tragelement (4) fest verbunden ist, wodurch sie mindestens einen Rohrabschnitt (49) definiert, der U-förmig gegenüber dem Tragelement (4) ist und dazu vorgesehen ist, während des Betriebs mit einer peristaltischen Pumpe (3) zusammenzuwirken.

30. Integriertes Modul nach Anspruch 29, wobei der mindestens eine U-förmige Rohrabschnitt (49) sich in Bezug auf die Umfangswand (32) des Tragelements (4) innen oder außen erstreckt.

## Revendications

1. Procédé de montage pour un module intégré (1) pour le traitement de fluide, comprenant les étapes suivantes:
- préparer un élément de support (4) pour un module intégré (1) pour le traitement de sang, comprenant:
○ un corps de base (6);
○ au moins un premier et au moins un deuxième connecteur (7) et (8) associés au corps de base (6) et placés à une certaine distance l'un de l'autre, aptes à recevoir et s'engager avec des contre-connecteurs correspondants (9) et (10) d'un dispositif de traitement du sang (5) pouvant être monté sur l'élément de support (4);
○ au moins un troisième connecteur (11) placé à une certaine distance dudit premier connecteur (7) et dudit deuxième connecteur (8) et relié directement au corps de base (6), lesdits premier, deuxième et troisième connecteurs (7), (8) et (11) définissant des paires de connecteurs ayant des entre-axes différents de l'un à l'autre pour s'engager à des paires correspondantes de contre-connecteurs (9) et (10) associés à plusieurs dispositifs de traitement du sang pouvant être montés sur l'élément de support (4);
- fixer un dispositif de traitement du sang (5) à l'élément de support (4);
- associer un circuit de distribution de fluide (15) à l'élément de support (4) et au dispositif de traitement du sang (5), **caractérisé en ce que** l'étape de fixer le dispositif de traitement du sang comprend les sous-étapes suivantes:
○ sélectionner une paire de connecteurs (7), (8), (11) auxquels les contre-connecteurs (9), (10) du dispositif de traitement du sang (5) sont à fixer;
○ appliquer une quantité préétablie de colle dans les sièges annulaires (21) de chaque connecteur ayant été sélectionné;
○ introduire au moins partiellement chaque contre-connecteur (9), (10) dans un siège annulaire respectif (21) afin d'obtenir un blocage mécanique et une étanchéité au liquide.

2. Procédé selon la revendication 1, où pendant ladite étape d'introduction au moins une partie de la quantité préétablie de colle est placée dans ladite deuxième zone (23) du siège annulaire respectif (21).

3. Procédé selon la revendication 2, où à la fin de ladite étape d'introduction une volume de ladite quantité préétablie de colle plus un volume de la portion de contre-connecteur (9), (10) reçue dans le siège annulaire (21) est inférieur à un volume total du siège annulaire.

4. Procédé selon la revendication 1, où l'étape d'associer un circuit de distribution de fluide (15) à l'élément de support (4) et au dispositif de traitement du sang (5) comprend les sous-étapes suivantes:
- fixer de façon étanche au liquide une portion d'extrémité d'un conduit de décharge (45) d'un fluide de décharge à la deuxième portion d'extrémité (14) de l'un desdits connecteurs (7), (8), (11), (26);
- fixer de façon étanche une portion d'extrémité d'un conduit d'alimentation de liquide de dialyse frais (48) à la deuxième portion d'extrémité (14) de l'autre desdits connecteurs (7), (8), (11), (26);
- fixer de façon étanche une portion d'extrémité d'une ramification de prélèvement de sang (46) à l'orifice d'entrée (42) du premier compartiment, et une portion d'extrémité d'une ramification de retour de sang (47) à l'orifice de sortie (43) du premier compartiment.

5. Module intégré (1) pour le traitement de fluide, comprenant:
- un élément de support (4) pour un module intégré (1) pour le traitement du sang, comprenant:
○ un corps de base (6);
○ au moins un premier et au moins un deuxième connecteur (7) et (8) associés au corps de base (6) et placés à une certaine distance l'un de l'autre, aptes à recevoir et s'engager avec des contre-connecteurs correspondants (9) et (10) d'un dispositif de traitement du sang (5) pouvant être monté sur l'élément de support (4);
○ au moins un troisième connecteur (11) placé à une certaine distance dudit premier connecteur (7) et dudit deuxième connecteur (8) et relié directement au corps de base (6), lesdits premier, deuxième et troisième connecteurs (7), (8) et (11) définissant des paires de connecteurs ayant des entre-axes différents de l'un à l'autre pour s'engager à des paires correspondantes de contre-connecteurs (9) et (10) associés à plusieurs dispositifs de traitement du sang pouvant être montés sur l'élément de support (4);
- au moins un dispositif de traitement du sang (5) engagé sur l'élément de support (4), ledit dispositif de traitement du sang (5) comprenant:
○ un corps contenant (40);
○ au moins une membrane semi-perméable (41) opérant à l'intérieur du corps contenant (40) et définissant un premier compartiment et un deuxième compartiment;
○ un premier contre-connecteur (9) et un deuxième contre-connecteur (10) associés au corps contenant (40) et fixés avec de la colle à une paire des trois connecteurs (7), (8) et (11) associés au corps de base (6), au moins un entre le premier contre-connecteur et le deuxième contre-connecteur étant mis en communication de fluide avec le deuxième compartiment du dispositif de traitement du sang (5) et avec des portions d'extrémité respectives (12) desdits connecteurs;
○ au moins un orifice d'entrée (42) au premier compartiment; et
○ au moins un orifice de sortie (43) du premier compartiment;
- un circuit de distribution de fluide (15) associé à l'élément de support (4) et coopérant avec le dispositif de traitement du sang (5).

6. Module intégré (1) selon la revendication 5, où les premier et deuxième connecteurs (7) et (8) sont liés directement au corps de base (6).

7. Module intégré (1) selon la revendication 6, où les premier et deuxième connecteurs (7) et (8) sont venus de construction avec le corps de base (6).

8. Module intégré (1) selon la revendication 5, où le troisième connecteur (11) est venu de construction avec le corps de base (6).

9. Module intégré (1) selon une quelconque des revendications précédentes 5 à 8, où chacun desdits connecteurs (7), (8) et (11) crée un passage de fluide ayant une première portion d'extrémité (12) apte à être mise en communication de fluide avec un canal correspondant (13) dans un contre-connecteur respectif (8) et (9) dans le dispositif de traitement du sang (5), et une deuxième portion d'extrémité (14) apte à être mise en communication de fluide avec un circuit de distribution de fluide (15) pouvant être associé au corps de base (6).

10. Module intégré (1) selon la revendication 9, où chacun desdits connecteurs (7), (8) et (11) comprend:
- un canal tubulaire (16) définissant ladite première portion d'extrémité (12),
- un collier d'étanchéité (17) placé dans une position radialement extérieure par rapport au canal tubulaire (16), et
- une paroi de liaison (18) se développant de façon continue entre une surface latérale extérieure (19) dudit canal tubulaire (16) et une surface latérale intérieure (20) dudit collier d'étanchéité (17) pour définir une siège annulaire (21) pour l'engagement de chaque contre-connecteur (9) et (10).

11. Module intégré (1) selon la revendication 10, où le canal tubulaire (16) définissant ladite première portion d'extrémité (12) est rangé de façon coaxiale par rapport au collier d'étanchéité (17), ledit siège annulaire (21) ayant un fond délimité par ladite paroi de liaison (18).

12. Module intégré (1) selon la revendication 11, où ledit siège annulaire (21) présente une dimension radiale augmentant progressivement dans une direction s'éloignant de ladite paroi de fond (18).

13. Module intégré (1) selon la revendication 12, où ledit siège annulaire (21) présente: une première zone (22) adjacente à ladite paroi de fond (18) et ayant une dimension radiale constante; une deuxième zone (23) distale par rapport à ladite paroi de fond (18) et ayant une dimension radiale constante supérieure à la dimension radiale de la première zone (22); et une troisième zone (24) qui est une zone de transition entre la première zone (22) et la deuxième zone (23) et présente une dimension qui augmente progressivement dans une direction s'éloignant de ladite paroi de fond (18).

14. Module intégré (1) selon la revendication 10, où le canal tubulaire (16) et le collier d'étanchéité (17) de chaque connecteur (7), (8) et (11) sont parallèles l'un à l'autre lorsqu'ils sortent du corps de base (6), définissant une seule direction pour l'accouplement avec des contre-connecteurs correspondants (9) et (10) d'un dispositif de traitement du sang (5).

15. Module intégré (1) selon la revendication 5, comprenant un quatrième connecteur (26) placé à une certaine distance desdits premier, deuxième et troisième connecteurs (7), (8) et (11), lequel quatrième connecteur (26) est venu de construction avec le corps de base (6) et définit avec au moins un entre lesdits premier, deuxième et troisième connecteurs (7), (8) et (11) une autre paire de connecteurs pouvant être engagée avec une paire correspondante de contre-connecteurs associés à un dispositif de traitement du sang pouvant être monté sur l'élément de support (4).

16. Module intégré (1) selon la revendication 15, où le quatrième connecteur (26) comprend:
- un corps de positionnement cylindrique central (27);
- un collier d'étanchéité (28) placé dans une position radialement extérieure par rapport au corps de positionnement cylindrique (27); et
- une paroi de liaison (29) se développant de façon continue entre une surface latérale extérieure (30) dudit corps de positionnement cylindrique (27) et une surface latérale intérieure (31) dudit collier d'étanchéité (28); ledit quatrième connecteur (26) définissant un point de liaison et d'étanchéité pour un contre-connecteur du dispositif de traitement du sang.

17. Module intégré (1) selon la revendication 6 ou 15, où lesdits connecteurs (7), (8), (11) et (26) et ledit corps de base (6) sont fabriqués dans un matériau rigide pour créer un support mécanique au dispositif de traitement du sang.

18. Module intégré (1) selon la revendication 15, où lesdits connecteurs (7), (8), (11) et (26) sont alignés l'un avec l'autre.

19. Module intégré (1) selon la revendication 6 ou 15, où lesdits connecteurs (7), (8), (11) et (26) sont rangés sur un côté du corps de base (6).

20. Module intégré (1) selon la revendication 5, où ledit corps de base (6) comprend une paroi antérieure (25) et une paroi périmétrale (32), laquelle paroi périmétrale (32) est reliée à travers l'un de ses côtés à la paroi antérieure (25) et définit une zone de travail (33) dans laquelle au moins une portion d'un circuit de distribution de fluide (15) apte à être associée à l'élément de support (4) peut être reçue.

21. Module intégré (1) selon la revendication 20, comprenant une structure auxiliaire (35) s'étendant latéralement et à l'extérieur par rapport à ladite zone de travail de logement (33) d'une zone de base (36) de la paroi périmétrale (32), lesdits connecteurs (7), (8), (11) et (26) saillant de ladite structure auxiliaire (35).

22. Module intégré (1) selon la revendication 5, où lesdits connecteurs (7), (8) et (11) ne sont pas alignés l'un avec l'autre.

23. Module intégré (1) selon la revendication 5, où le corps de base (6) comprend une paroi antérieure (25) de laquelle lesdits connecteurs (7), (8) et (11) saillissent directement, et une couverture (39) associée à une paroi périmétrale (32) sur un bord opposé par rapport à la paroi antérieure (25).

24. Module intégré selon une quelconque des revendications précédentes 5 à 23, où ledit dispositif de traitement du sang (5) est fixé au corps de base (6) par au moins une paire desdits connecteurs (7), (8) et (11).

25. Module intégré selon la revendication 5, où ladite paire de connecteurs est placée entre les contre-connecteurs (9) et (10) et une portion du circuit de distribution de fluide (15).

26. Module intégré selon la revendication 5, où le circuit de distribution de fluide (15) comprend au moins un conduit de décharge (45) d'un fluide de décharge, mis en communication avec la deuxième portion d'extrémité (14) de l'un desdits connecteurs (7), (8) et (11).

27. Module intégré selon la revendication 5, où le circuit de distribution de fluide (15) comprend au moins un conduit d'alimentation de liquide de dialyse frais (48), mis en communication avec la deuxième portion d'extrémité (14) de l'autre desdits connecteurs (7), (8) et (11).

28. Module intégré selon une quelconque des revendications précédentes 5 à 27, où le circuit de distribution de fluide (15) comprend au moins un conduit de circuit du sang (44) ayant une ramification de prélèvement du sang (46), mise en communication avec l'orifice d'entrée (42) du premier compartiment, et au moins une ramification de retour du sang (47), mise en communication avec l'orifice de sortie (43) du premier compartiment.

29. Module selon une quelconque des revendications 26, 27 ou 28, où au moins un desdits conduits (44), (45) et (48) est lié à l'élément de support (4), définissant ainsi au moins une portion de conduit (49) en forme de U par rapport à l'élément de support (4) et apte pendant l'usage à coopérer avec une pompe péristaltique (3).

30. Module intégré selon la revendication 29, où l'au moins une portion de conduit en forme de U (49) s'étend à l'intérieur ou à l'extérieur par rapport à la paroi périmétrale (32) de l'élément de support (4).
